(19) Europäisches Patentamt / European Patent Office / Office européen des brevets

(11) **EP 4 800 034 A1**

(12) **EUROPEAN PATENT APPLICATION**
published in accordance with Art. 153(4) EPC


(43) Date of publication:
**02.09.2026 Bulletin 2026/36**

(21) Application number: **24882491.4**

(22) Date of filing: **25.10.2024**

(51) International Patent Classification (IPC):
***C07K 16/28*** (2006.01) ***A61K 39/395*** (2006.01)
***A61K 51/10*** (2006.01) ***A61P 35/00*** (2006.01)
***C12N 5/20*** (2006.01) ***C12N 15/13*** (2006.01)
***C12P 21/08*** (2006.01) ***A61K 103/40*** (2006.01)

(52) Cooperative Patent Classification (CPC):
**A61K 39/395; A61K 51/10; A61P 35/00; C07K 16/00; C07K 16/28; C12N 5/163;** A61K 51/00

(86) International application number:
**PCT/JP2024/038102**

(87) International publication number:
**WO 2025/089383 (01.05.2025 Gazette 2025/18)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: **26.10.2023 JP 2023184107**



(71) Applicant: **Perseus Proteomics Inc.**
**Tokyo 103-0015 (JP)**

(72) Inventors:
- **KAJITA, Masamichi**
**Tokyo 104-0031 (JP)**
- **HABA, Hiromitsu**
**Wako-shi, Saitama 351-0198 (JP)**

(74) Representative: **Plougmann Vingtoft a/s**
**Strandvejen 70**
**2900 Hellerup (DK)**


Remarks:
The complete document including Reference Table(s) and the Sequence Listing(s) can be downloaded from the EPO website

(54) **225AC-LABELED ANTI-CDH3 ANTIBODIES**


(57) An object of the present invention is to provide a radiolabeled anti-CDH3 antibody exhibiting excellent antitumor efficacy, and a cancer treatment agent containing the same. The present invention relates to an $^{225}$Ac-labeled anti-CDH3 antibody and a cancer treatment agent containing the same.


EP 4 800 034 A1

## Description

Technical Field

The present invention relates to an $^{225}$Ac-labeled anti-CDH3 antibody and a cancer treatment agent containing the same.

Background Art

An anti-CDH3 antibody labeled with $^{90}$Y ($^{90}$Y-labeled anti-CDH3 antibody) is expected to be useful as a treatment agent for CDH3-expressing tumors (PTL 1). Then, a $^{90}$Y-labeled anti-CDH3 antibody found by the applicant exhibits excellent antitumor efficacy in studies using cancer-bearing mice (PTL 2), and safety and efficacy of $^{90}$Y-FF-21101, which is a candidate product of the $^{90}$Y-labeled anti-CDH3 antibody as a drug, were evaluated in a clinical trial conducted on humans (NPL 1). FF-21101 is a chimeric anti-CDH3 monoclonal antibody into which DOTA is introduced with isothiocyanobenzyl-DOTA, and the anti-CDH3 monoclonal antibody has a heavy chain of the amino acid sequence of SEQ ID NO:1 and a light chain of the amino acid sequence of SEQ ID NO:2.

Therapeutic nuclides other than $^{90}$Y include $^{59}$Fe, $^{67}$Cu, $^{89}$Sr, $^{103}$Ru, $^{153}$Sm, $^{165}$Dy, $^{166}$Ho, $^{177}$Lu, $^{186}$Re, $^{188}$Re, $^{198}$Au, $^{211}$At, $^{223}$Ra, $^{224}$Ra, $^{226}$Th, $^{227}$Th, $^{230}$U, $^{149}$Tb, $^{212}$Bi, $^{213}$Bi, $^{225}$Ac, $^{212}$Pb, and the like; however, there is no example in which an anti-CDH3 antibody is labeled therewith, and effectiveness thereof is examined.

Citation List

Patent Literature

PTL 1: JP5593560B
PTL 2: JP5380553B

Non Patent Literature

NPL 1: Clinical Cancer Research, 2020 Nov. 15; 26(22):5830-5842

Summary of Invention

Technical Problem

Although a $^{90}$Y-labeled anti-CDH3 antibody exhibits excellent antitumor efficacy, there remains room for improvement in pursuing further enhanced efficacy, and it has remained unknown whether antitumor efficacy superior to that of $^{90}$Y-labeled anti-CDH3 antibodies is achieved by using an anti-CDH3 antibody labeled with a different radionuclide.

The present invention was made in view of the above circumstances, and an object thereof is to provide a radionuclide-labeled anti-CDH3 antibody exhibiting excellent antitumor efficacy, and a cancer treatment agent containing the same as an active ingredient.

Solution to Problem

The present inventors prepared an anti-CDH3 antibody labeled with $^{225}$Ac and examined antitumor efficacy thereof, found that the antitumor efficacy of the $^{225}$Ac-labeled anti-CDH3 antibody is significantly superior to that of $^{90}$Y-labeled anti-CDH3 antibodies, and thus completed the present invention.

That is, the present invention provides the following items [1] to [22].

[1] An $^{225}$Ac-labeled anti-CDH3 antibody in which $^{225}$Ac binds to an anti-CDH3 antibody or a recombinant antibody thereof, the anti-CDH3 antibody containing:

a heavy-chain variable region containing CDR1 that contains an amino acid sequence of SEQ ID NO:3, CDR2 that contains an amino acid sequence of SEQ ID NO:4, and CDR3 that contains an amino acid sequence of SEQ ID NO:5; and

a light-chain variable region containing CDR1 that contains an amino acid sequence of SEQ ID NO:6, CDR2 that contains an amino acid sequence of SEQ ID NO:7, and CDR3 that contains an amino acid sequence of SEQ ID NO:8.

[2] The $^{225}$Ac-labeled anti-CDH3 antibody described in item [1], in which the anti-CDH3 antibody contains:

a heavy-chain variable region containing an amino acid sequence of SEQ ID NO:9; and
a light-chain variable region containing an amino acid sequence of SEQ ID NO:10.

[3] The $^{225}$Ac-labeled anti-CDH3 antibody described in item [1] or [2], in which the anti-CDH3 antibody contains:

a heavy chain containing an amino acid sequence of SEQ ID NO:1; and
a light chain containing an amino acid sequence of SEQ ID NO:2.

[4] The $^{225}$Ac-labeled anti-CDH3 antibody described in any of items [1] to [3], in which the anti-CDH3 antibody is a monoclonal antibody produced by an antibody-producing cell with Accession No. NITE BP-1040 or Accession No. NITE BP-1049.
[5] The $^{225}$Ac-labeled anti-CDH3 antibody described in item [1], in which the anti-CDH3 antibody or the recombinant antibody thereof binds to the $^{225}$Ac via a metal chelate.
[6] The $^{225}$Ac-labeled anti-CDH3 antibody described in item [5], in which the metal chelate is DOTA.
[7] A cancer treatment agent containing the $^{225}$Ac-labeled anti-CDH3 antibody described in item [1].
[8] The $^{225}$Ac-labeled anti-CDH3 antibody described in item [1], which is for use in a cancer treatment.
[9] Use of the $^{225}$Ac-labeled anti-CDH3 antibody described in item [1] for a production of a cancer treatment agent.
[10] A method for treating a cancer, including administering the $^{225}$Ac-labeled anti-CDH3 antibody described in item [1], to a subject in need thereof.
[11] The cancer treatment agent described in item [7], in which a disease to be treated is a pancreatic cancer.
[12] The cancer treatment agent described in item [7], in which a dose for administration to a human is 0.10 to 10 $MBq/m^2$.
[13] The cancer treatment agent described in item [7], in which specific radioactivity of the $^{225}$Ac-labeled anti-CDH3 antibody is 0.01 to 5.0 MBq/mg.
[14] The $^{225}$Ac-labeled anti-CDH3 antibody described in item [8], in which a disease to be treated is a pancreatic cancer.
[15] The $^{225}$Ac-labeled anti-CDH3 antibody described in item [8], in which a dose for administration to a human is 0.10 to 10 $MBq/m^2$.
[16] The $^{225}$Ac-labeled anti-CDH3 antibody described in item [8], in which specific radioactivity of the $^{225}$Ac-labeled anti-CDH3 antibody is 0.01 to 5.0 MBq/mg.
[17] The use of the $^{225}$Ac-labeled anti-CDH3 antibody described in item [9], in which a disease to be treated is a pancreatic cancer.
[18] The use of the $^{225}$Ac-labeled anti-CDH3 antibody described in item [9], in which a dose for administration to a human is 0.10 to 10 $MBq/m^2$.
[19] The use of the $^{225}$Ac-labeled anti-CDH3 antibody described in item [9], in which specific radioactivity of the $^{225}$Ac-labeled anti-CDH3 antibody is 0.01 to 5.0 MBq/mg.
[20] The method for treating a cancer described in item [10], in which a disease to be treated is a pancreatic cancer.
[21] The method for treating a cancer described in item [10], in which a dose for administration to a human is 0.10 to 10 $MBq/m^2$.
[22] The method for treating a cancer described in item [10], in which specific radioactivity of the $^{225}$Ac-labeled anti-CDH3 antibody is 0.01 to 5.0 MBq/mg.

Advantageous Effects of Invention

An $^{225}$Ac-labeled anti-CDH3 antibody of the present invention exhibits excellent antitumor efficacy and is useful as a cancer treatment agent.

Brief Description of Drawings

[FIG. 1] FIG. 1 is a diagram illustrating biological distribution of $^{225}$Ac-labeled FF-21101 in HPAF-II-transplanted model

mice.

[FIG. 2] FIG. 2 is a diagram illustrating temporal changes in tumor volume in HPAF-II-transplanted model mice in a group subjected to administration of PBS.

[FIG. 3] FIG. 3 is a diagram illustrating temporal changes in tumor volume in HPAF-II-transplanted model mice in a group subjected to administration of $^{225}$Ac-labeled FF-21101 (0.71 MBq/kg).

[FIG. 4] FIG. 4 is a diagram illustrating temporal changes in tumor volume in HPAF-II-transplanted model mice in a group subjected to administration of $^{225}$Ac-labeled FF-21101 (1.07 MBq/kg).

[FIG. 5] FIG. 5 is a diagram illustrating temporal changes in tumor volume in HPAF-II-transplanted model mice in a group subjected to administration of $^{225}$Ac-labeled FF-21101 (1.42 MBq/kg).

[FIG. 6] FIG. 6 is a diagram illustrating body weight change ratios of HPAF-II-transplanted model mice in the group subjected to administration of PBS.

[FIG. 7] FIG. 7 is a diagram illustrating body weight change ratios of HPAF-II-transplanted model mice in the group subjected to administration of $^{225}$Ac-labeled FF-21101 (0.71 MBq/kg).

[FIG. 8] FIG. 8 is a diagram illustrating body weight change ratios of HPAF-II-transplanted model mice in the group subjected to administration of $^{225}$Ac-labeled FF-21101 (1.07 MBq/kg).

[FIG. 9] FIG. 9 is a diagram illustrating body weight change ratios of HPAF-II-transplanted model mice in the group subjected to administration of $^{225}$Ac-labeled FF-21101 (1.42 MBq/kg).

[FIG. 10] FIG. 10 is a diagram illustrating temporal changes in tumor volume in HPAF-II-transplanted model mice in a group subjected to administration of PBS.

[FIG. 11] FIG. 11 is a diagram illustrating temporal changes in tumor volume in HPAF-II-transplanted model mice in a group subjected to administration of $^{90}$Y-labeled FF-21101 (308 MBq/kg).

[FIG. 12] FIG. 12 is a diagram illustrating body weight change ratios of HPAF-II-transplanted model mice in the group subjected to administration of PBS.

[FIG. 13] FIG. 13 is a diagram illustrating body weight change ratios of HPAF-II-transplanted model mice in the group subjected to administration of $^{90}$Y-labeled FF-21101 (308 MBq/kg).

Description of Embodiments

An $^{225}$Ac-labeled anti-CDH3 antibody of the present invention is an $^{225}$Ac-labeled anti-CDH3 antibody in which $^{225}$Ac binds to an anti-CDH3 antibody. A "bond" herein means a single chemical bond or a plurality of chemical bonds, and the anti-CDH3 antibody and $^{225}$Ac may be linked via a covalent bond and a coordination bond in the $^{225}$Ac-labeled anti-CDH3 antibody of the present invention. The bond with $^{225}$Ac is typically a metal chelate, and the metal chelate covalently binds to the anti-CDH3 antibody. That is, the anti-CDH3 antibody binds to $^{225}$Ac via the metal chelate.

The anti-CDH3 antibody used in the present invention is an anti-CDH3 antibody or a recombinant antibody thereof, the anti-CDH3 antibody containing: a heavy-chain variable region containing CDR1 that contains an amino acid sequence of SEQ ID NO:3, CDR2 that contains an amino acid sequence of SEQ ID NO:4, and CDR3 that contains an amino acid sequence of SEQ ID NO:5; and a light-chain variable region containing CDR1 that contains an amino acid sequence of SEQ ID NO:6, CDR2 that contains an amino acid sequence of SEQ ID NO:7, and CDR3 that contains an amino acid sequence of SEQ ID NO:8.

Among such antibodies, an anti-CDH3 antibody containing: a heavy-chain variable region containing an amino acid sequence of SEQ ID NO:9; and a light-chain variable region containing an amino acid sequence of SEQ ID NO:10, or a recombinant antibody of the anti-CDH3 antibody is preferable, and an anti-CDH3 antibody containing: a heavy-chain containing an amino acid sequence of SEQ ID NO:1; and a light-chain containing an amino acid sequence of SEQ ID NO:2, or a recombinant antibody of the anti-CDH3 antibody is more preferable. These antibodies are described in PTL 2 and can be produced or available according to the methods described in PTL 2, for example.

In addition, among the anti-CDH3 antibodies described in PTL 2, an antibody produced by an antibody-producing cell with Accession No. NITE BP-1040 or Accession No. NITE BP-1049, or a recombinant antibody of the anti-CDH3 antibody is a preferable anti-CDH3 antibody, and an antibody produced by the antibody-producing cell with Accession No. NITE BP-1049, or a recombinant antibody of the anti-CDH3 antibody is particularly preferable.

A recombinant antibody can be produced using a known method. A chimeric antibody is an antibody containing: a heavy-chain variable region and light-chain variable region of a non-human mammal antibody such as a mouse antibody; and a heavy-chain constant region and light-chain constant region of a human antibody, and can be obtained by linking DNA encoding the variable regions of the mouse antibody to DNA encoding the constant regions of the human antibody, incorporating the same into an expression vector, introducing the same into a host, and producing the chimeric antibody. A humanized antibody is also referred to as a reshaped human antibody, and is an antibody in which a complementarity determining region of a non-human mammal antibody such as a mouse antibody is transplanted onto a complementarity determining region of a human antibody, and a general genetic engineering method therefor is also known. Specifically, the humanized antibody is synthesized by a PCR method from several oligonucleotides produced such that DNA sequences,

which are designed to link a CDR of a mouse antibody and a framework region of a human antibody, have an overlapping portion at a terminal portion. The obtained DNA is linked to DNA encoding the constant regions of the human antibody, subsequently incorporating the same into an expression vector, introducing the same into a host, and producing the humanized antibody. A region allowing a complementarity determining region to form a favorable antigen-binding site is selected as the framework region of the human antibody linked via the complementarity determining region.

A method for producing a chimeric antibody or a humanized antibody using a genetic engineering method is known. That is, confirmed VH and VL sequences of a parental monoclonal antibody are altered through genetic engineering, followed by chimerization or humanization by a usual method.

Examples of the metal chelate capable of binding to $^{225}$Ac used in the present invention include DOTA, CHX-DTPA, MX-DTPA, HEHA, PEPA, TETA, TEPTA, DOTPA, DOTMP, macropa, macropid, bispa2, octopa, and crown. Among these metal chelates, DOTA is preferable. Binding between the anti-CDH3 antibody and the metal chelate is achieved according to the methods described in PTL 2 or a commonly used method. For example, amino acids constituting an antibody have various types of side chains, and a metal chelate into which a substituent capable of forming a bond with these side chains under mild conditions is introduced is known. As such a substituent, a carboxylic acid, an NHS ester, a TFP ester, an isothiocyanate, a maleimide, an alkyne, and a tetrazine, and the like are known, and the substituent binds to a primary amine, thiol, azide, trans-cyclooctene, or the like of an antibody to form a covalent bond. As a metal chelate into which such a substituent is introduced, isothiocyanobenzyl-DOTA is known and widely used (Chemistry Society Reviews, 2014, 43, 260-290).

The $^{225}$Ac-labeled anti-CDH3 antibody of the present invention may be synthesized by reacting $^{225}$Ac and the anti-CDH3 antibody into which the metal chelate is introduced. $^{225}$Ac can be obtained through a known nuclear reaction and purification; however, commercially available $^{225}$Ac can also be used. Commercially available $^{225}$Ac may be obtained as a solid nitrate salt, and when $^{225}$Ac is reacted with an antibody, $^{225}$Ac may be dissolved with an acid or a pH buffer solution to prepare an acidic aqueous solution and used. Specifically, the anti-CDH3 antibody to which the metal chelate is introduced and $^{225}$Ac may be dissolved in an aqueous solution with a pH value adjusted to 4 to 6, and subjected to reaction under a heating condition of 25°C to 45°C for 15 to 120 minutes.

The $^{225}$Ac-labeled anti-CDH3 antibody of the present invention is preferably an $^{225}$Ac-labeled anti-CDH3 antibody in which an anti-CDH3 antibody or a recombinant antibody thereof binds to $^{225}$Ac via DOTA, the anti-CDH3 antibody containing: a heavy-chain variable region containing CDR1 that contains an amino acid sequence of SEQ ID NO:3, CDR2 that contains an amino acid sequence of SEQ ID NO:4, and CDR3 that contains an amino acid sequence of SEQ ID NO:5; and a light-chain variable region containing CDR1 that contains an amino acid sequence of SEQ ID NO:6, CDR2 that contains an amino acid sequence of SEQ ID NO:7, and CDR3 that contains an amino acid sequence of SEQ ID NO:8.

The $^{225}$Ac-labeled anti-CDH3 antibody of the present invention is preferably an $^{225}$Ac-labeled anti-CDH3 antibody in which an anti-CDH3 antibody or a recombinant antibody thereof binds to $^{225}$Ac via DOTA, the anti-CDH3 antibody containing: a heavy-chain variable region containing an amino acid sequence of SEQ ID NO:9; and a light-chain variable region containing an amino acid sequence of SEQ ID NO:10.

The $^{225}$Ac-labeled anti-CDH3 antibody of the present invention is preferably an $^{225}$Ac-labeled anti-CDH3 antibody in which an anti-CDH3 antibody or a recombinant antibody thereof binds to $^{225}$Ac via DOTA, the anti-CDH3 antibody containing: a heavy-chain containing an amino acid sequence of SEQ ID NO:1; and a light-chain containing an amino acid sequence of SEQ ID NO:2.

The $^{225}$Ac-labeled anti-CDH3 antibody of the present invention is preferably an $^{225}$Ac-labeled anti-CDH3 antibody in which a monoclonal antibody produced by an antibody-producing cell with Accession No. NITE BP-1040 or Accession No. NITE BP-1049, or a recombinant antibody of the anti-CDH3 antibody binds to $^{225}$Ac via DOTA.

A cancer treatment agent of the present invention contains the $^{225}$Ac-labeled anti-CDH3 antibody as an active ingredient. The anti-CDH3 antibody delivers $^{225}$Ac to CDH3-expressing tumor cells, and $^{225}$Ac exerts the efficacy thereof through cell-damaging activity of alpha radiation generated from $^{225}$Ac.

A target of the cancer treatment agent of the present invention is a CDH3-expressing tumor, and target diseases include cancers. Although the type of cancer is not particularly limited, examples thereof include lung cancer, ovarian cancer, pancreatic cancer, triple-negative breast cancer, head and neck cancer, bile duct cancer, and colorectal cancer, and among them, pancreatic cancer is preferable.

The cancer treatment agent of the present invention can be used to suppress a cancer by administering an effective amount of the $^{225}$Ac-labeled anti-CDH3 antibody to a mammal including a human. "Suppression of a cancer" has the broadest meaning including both: preventive effects such as prevention of tumor development, metastasis, trans-plantation, and recurrence; and therapeutic effects such as suppression of tumor cell proliferation, inhibition of tumor growth by reducing tumor size, and amelioration of symptoms, for example, and is not intended to be construed in a limiting manner in any case.

The cancer treatment agent of the present invention may contain an unlabeled anti-CDH3 antibody. This is attributable to the difference in the amounts of substances in a reaction for labeling an antibody with a radionuclide. This is

because the antibody is usually reacted in an amount of substance that is orders of magnitude greater than the amount of substance of the radionuclide, and only a very small amount of the antibody is labeled with the radionuclide. Accordingly, a large amount of the antibody unlabeled remains in the reaction solution; however, the unlabeled antibody is usually not separated from the labeled antibody and forms part of the cancer treatment agent. Note that the amount of radioactivity per unit mass of an antibody is defined as specific radioactivity (such as MBq/$\mu$g) and may serve as a factor for controlling pharmacokinetics of an active ingredient.

The specific radioactivity of the $^{225}$Ac-labeled anti-CDH3 antibody contained in the cancer treatment agent of the present invention may be set so as to reduce side effects within a range in which antitumor efficacy is obtained, may be 0.01 to 5.0 MBq/mg, is preferably 0.1 to 3.0 MBq/mg, and is particularly preferably 0.5 to 1.5 MBq/mg, for example.

The cancer treatment agent of the present invention may be prepared as an aqueous solution, preferably an aqueous solution in which the $^{225}$Ac-labeled anti-CDH3 antibody is dissolved in physiological sodium chloride solution. A pharmaceutically acceptable additive may be usually mixed, as appropriate. Examples of the additive include a stabilizer, a pH modifier, a tonicity agent, a solubilizer, and a chelating agent.

Examples of the stabilizer include gentisic acid, ascorbic acid, benzyl alcohol, polyvinylpyrrolidone, and ethanol.

Examples of the pH modifier include hydrochloric acid, sodium hydroxide, an acetic acid buffer, a benzoic acid buffer, and a histidine buffer.

Examples of the tonicity agent include sodium chloride and glucose.

Examples of the solubilizer include Polysorbate 80.

Examples of the chelating agent include ethylenediaminetetraacetic acid, diethylenetriaminepentaacetic acid, hexaethyleneheptamine, and 1,4,7,10-tetraazacyclododecane-1,4,7,10-tetraacetic acid.

Any one kind of these additives may be used, or two or more kinds thereof may be used in combination.

Blending amounts of these additives are not particularly limited, and the additives may be appropriately blended in accordance with each purpose so that effects thereof are sufficiently exerted.

The cancer treatment agent of the present invention can be administered in a form of an injectable preparation, and is administered to a patient through intravenous administration, topical administration, intraperitoneal administration, hepatic arterial infusion, or the like. In addition, an administration method, dosage amount, and administration frequency may be appropriately selected according to the age, body weight, and condition of a patient.

Although the dosage amount varies depending on the condition, body weight, age, and the like of a patient, a single dosage amount for an adult may be 0.10 to 10 MBq/m$^2$, is preferably 1.0 to 7.0 MBq/m$^2$, and is particularly preferably 2.0 to 4.5 MBq/m$^2$, for example. The dosage amount for humans can also be determined through conversion based on a result of a dosage amount study on mice. A dosage amount (MBq/kg) for mice is set, and the dosage amount for humans (MBq/m$^2$) may be set by multiplying the mouse dosage amount by a Mouse $K_m$ factor (3 kg/m$^2$, J Basic Clin Pharm., 2016 Mar; 7(2): 27-31). The dosage amount may be set so that optimal antitumor efficacy is obtained within a range in which side effects are acceptable.

## Examples

Next, the present invention will be described in more detail with reference to Examples; however, the present invention is not limited thereto.

### Example 1: preparation of $^{225}$Ac-labeled FF-21101

An $^{225}$Ac solution in which an $^{225}$Ac nitrate salt (Oak Ridge National Laboratory) was dissolved in 0.05 mol/L of HCl was added to an aqueous solution (0.25M sodium acetate, pH: 5.5) of 5 mg/mL of FF-21101 (FUJIFILM Diosynth Biotechnologies U.S.A., Inc.), followed by incubation at 45°C for 1 hour.

A portion of the labelling reaction solution was sampled, and a labeling yield was confirmed using thin-layer chromatography (manufactured by Merck & Co., Inc.) with 10 mmol/L DTPA/physiological sodium chloride solution as a developing solvent. The radioactivity at a peak in the vicinity of the original position of the strip and the radioactivity over the entire strip development range were measured using a radiochromatogram scanner, and the labeling yield was calculated according to the following formula and was confirmed to be 90% or more. Note that in this measurement system, free $^{225}$Ac ions migrate toward the solvent front, while the labeled compound remains at the original position.

Labeling yield (%) = radioactivity at vicinity of original position/radioactivity over entire development range $\times$ 100

### Comparative Example 1: preparation of $^{90}$Y-labeled FF-21101

A $^{90}$Y solution (Eckert & Ziegler) was added to an aqueous solution (0.25M sodium acetate, pH: 5.5) of 5 mg/mL of FF-21101 (FUJIFILM Diosynth Biotechnologies U.S.A., Inc.), followed by incubation at 40°C for 15 minutes.

A portion of the labelling reaction solution was sampled, and a labeling yield was confirmed using thin-layer chromatography (manufactured by Biodex Medical Systems, Inc.) with 10 mmol/L DTPA/physiological sodium chloride solution as a developing solvent. The radioactivity at a peak in the vicinity of the original position of the strip and the radioactivity over the entire strip development range were measured using a radiochromatogram scanner, and the labeling yield was calculated according to the following formula and was confirmed to be 90% or more. Note that in this measurement system, free $^{90}$Y ions migrate toward the solvent front, while the labeled compound remains at the original position.

Labeling yield (%) = radioactivity at vicinity of original position/radioactivity over entire development range × 100

Example 2: confirmation of maximum tolerable dose of $^{225}$Ac-labeled FF-21101 in cancer-bearing mice

Human pancreatic cancer-derived cell line HPAF-II (ATCC) ($5 \times 10^6$ cells) was subcutaneously transplanted into the right thoracic region of 6-week-old BALB/cSlc-nu/nu mice (female), and nine days after cell transplantation, $^{225}$Ac-labeled FF-21101 (administered radioactivity: 1.07, 1.42, or 2.13 MBq/kg, antibody dosage amount: 1.04 mg/kg) was administered, followed by an 18-day observation (n = 5 per group). In the 2.13 MBq/kg group, two mice exhibited a decrease in body weight to below 80% of the body weight at the time of cell transplantation or at the start of administration, whereas no decrease was observed in the other groups. Accordingly, 1.42 MBq/kg was determined to be the maximum tolerable dose.

Example 3: biological distribution of $^{225}$Ac-labeled FF-21101 in cancer-bearing mice

Human pancreatic cancer-derived cell line HPAF-II (ATCC) ($5 \times 10^6$ cells) was subcutaneously transplanted into the right thoracic region of 6-week-old BALB/cSlc-nu/nu mice (female), and nine days after cell transplantation, $^{225}$Ac-labeled FF-21101 (administered radioactivity: 0.71 MBq/kg, antibody dosage amount: 1.04 mg/kg) was administered. At 5 minutes and 24, 48, 96, and 168 hours after administration, blood, heart, lung, liver, kidney, spleen, pancreas, bone, and tumor samples were collected, and the radioactive concentration per unit weight (%ID/g) was calculated (n = 3, provided that n = 2 for blood at 5 minutes after administration).

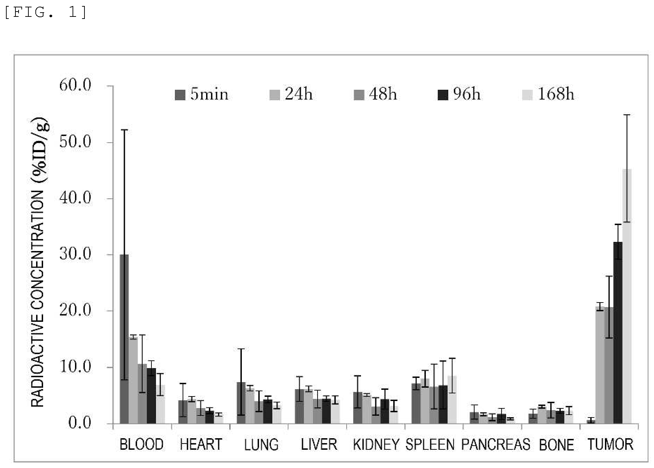

Table 1 and Table 2 show the mean (%ID/g) and the standard deviation (%ID/g) in each organ, respectively. In addition, FIG. 1 shows graphical representations thereof. At 168 hours after administration, it could be confirmed that a high tumor uptake with a mean value of 45.4 %ID/g was achieved.

[Table 1]

| Time point | Blood | Heart | Lung | Liver | Kidney | Spleen | Pancreas | Bone | Tumor |
|---|---|---|---|---|---|---|---|---|---|
| 5min | 30.0 | 4.2 | 7.4 | 6.2 | 5.7 | 7.2 | 2.1 | 1.8 | 0.6 |
| 24h | 15.3 | 4.4 | 6.4 | 6.3 | 5.2 | 8.1 | 1.7 | 3.1 | 20.8 |
| 48h | 10.7 | 2.8 | 4.0 | 4.4 | 3.1 | 6.6 | 1.2 | 2.4 | 20.7 |
| 96h | 9.9 | 2.4 | 4.4 | 4.5 | 4.4 | 6.9 | 1.7 | 2.3 | 32.3 |
| 168h | 7.0 | 1.6 | 3.3 | 4.3 | 3.2 | 8.6 | 0.9 | 2.3 | 45.4 |

[Table 2]

| Time point | Blood | Heart | Lung | Liver | Kidney | Spleen | Pancreas | Bone | Tumor |
|---|---|---|---|---|---|---|---|---|---|
| 5min | 22.2 | 3.0 | 5.9 | 2.2 | 2.8 | 1.1 | 1.3 | 0.8 | 0.5 |
| 24h | 0.4 | 0.5 | 0.5 | 0.5 | 0.3 | 1.5 | 0.3 | 0.3 | 0.7 |
| 48h | 5.0 | 1.3 | 1.9 | 1.6 | 1.6 | 4.0 | 0.6 | 1.4 | 5.5 |
| 96h | 1.3 | 0.5 | 0.6 | 0.5 | 1.8 | 4.3 | 1.0 | 0.4 | 3.1 |
| 168h | 1.9 | 0.3 | 0.6 | 0.7 | 1.0 | 3.1 | 0.2 | 0.7 | 9.5 |

Example 4: antitumor efficacy of $^{225}$Ac-labeled FF-21101 in cancer-bearing mice

Human pancreatic cancer-derived cell line HPAF-II (ATCC) ($5 \times 10^6$ cells) was subcutaneously transplanted into the right thoracic region of 7-week-old BALB/cSlc-nu/nu mice (female), and eight days after cell transplantation, $^{225}$Ac-

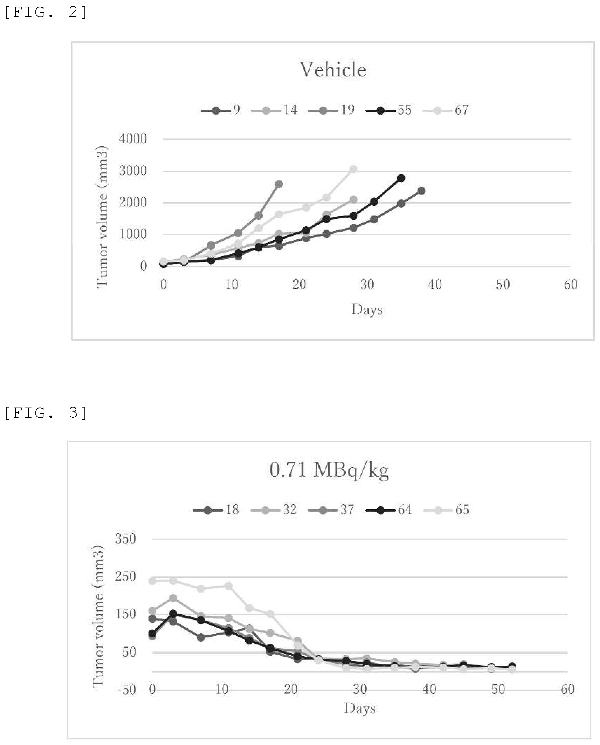

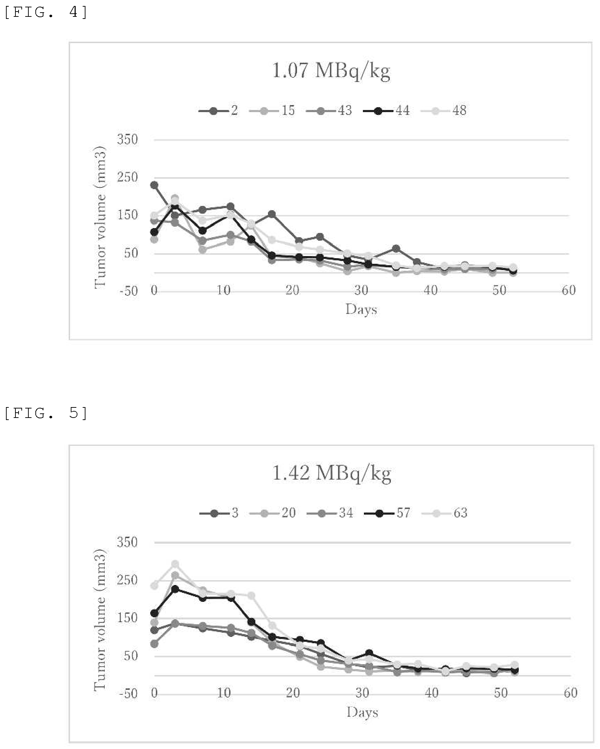

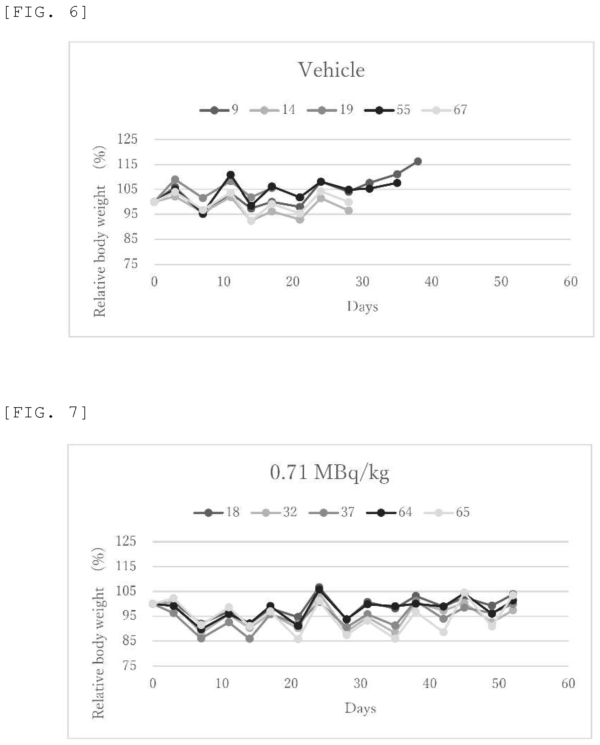

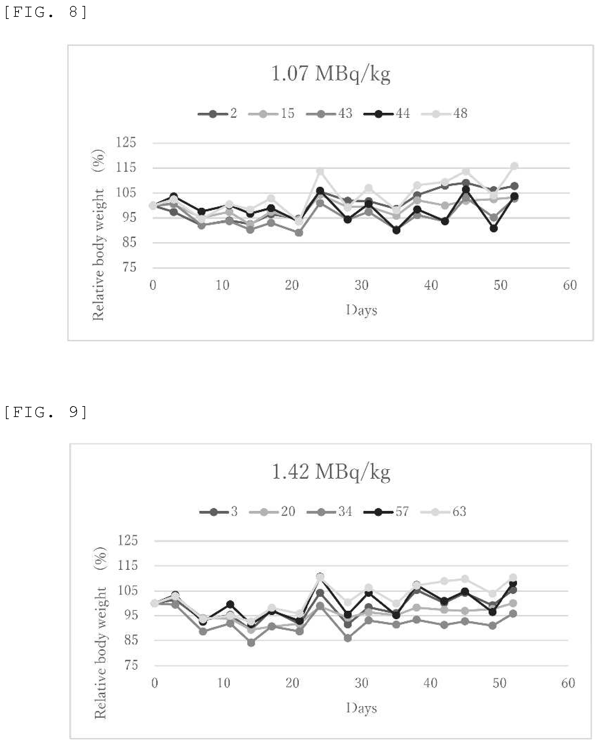

labeled FF-21101 (administered radioactivity: 0.71, 1.07, or 1.42 MBq/kg, antibody dosage amount: 1.04 mg/kg) or PBS (vehicle) was administered, and the tumor volume and the body weight were measured (n = 5 per group). The temporal changes in the tumor volume are shown in FIG. 2 to FIG. 5, and the temporal changes in the body weight are shown in FIG. 6 to FIG. 9. Whereas the tumor volume increased in the PBS group, tumor growth was suppressed in the $^{225}$Ac-labeled FF-21101 groups at all administered radioactivity levels up to 52 days after administration, and no mice exhibited a decrease in body weight to below 80% of the body weight at the start of administration.

Comparative Example 2: antitumor efficacy of $^{90}$Y-labeled FF-21101 in cancer-bearing mice

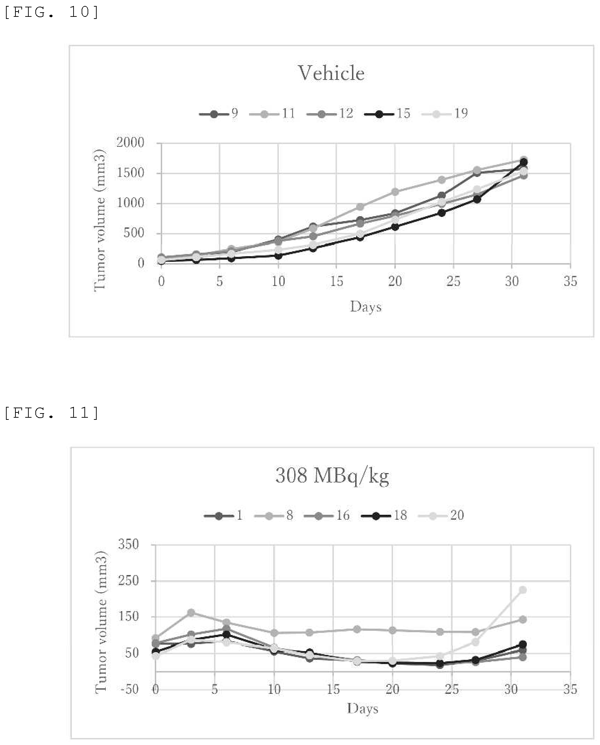

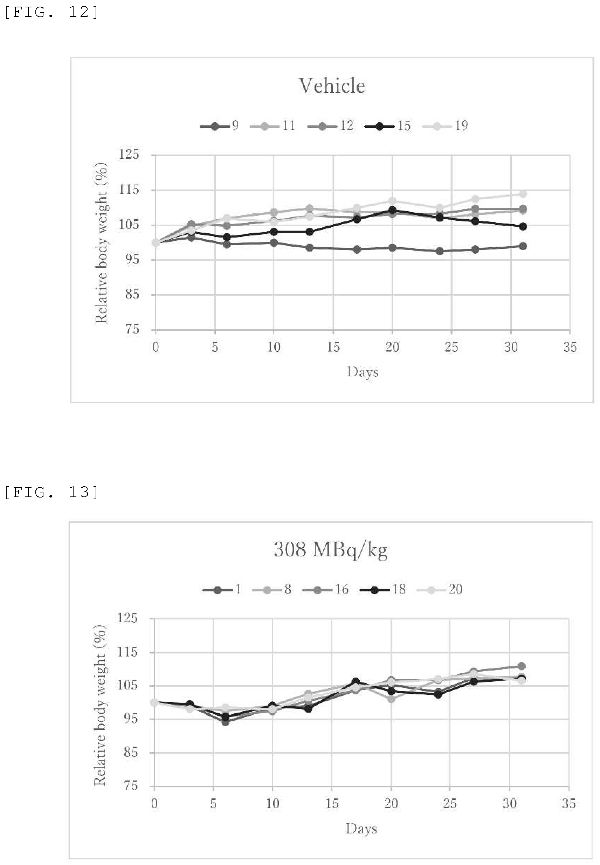

Human pancreatic cancer-derived cell line HPAF-II (ATCC) ($5 \times 10^6$ cells) was subcutaneously transplanted into the right thoracic region of 7-week-old BALB/cSlc-nu/nu mice (female), and eight days after cell transplantation, $^{90}$Y-labeled FF-21101 (308 MBq/kg, 1.04 mg/kg) or PBS (vehicle) was administered, and the tumor volume and the body weight were measured (n = 5 per group). Note that the administration dosage 308 MBq/kg was set based on conversion of the clinical dose of 925 MBq/m$^2$ (NPL 1) of $^{90}$Y-labeled FF-21101 into a dose per body weight of a mouse. The temporal changes in the tumor volume are shown in FIG. 10 and FIG. 11, and the temporal changes in the body weight are shown in FIG. 12 and FIG. 13. Whereas the tumor volume increased in the PBS group, in the $^{90}$Y-labeled FF-21101 group, although tumor growth was temporally suppressed, a tendency toward regrowth was observed. In addition, no mice exhibited a decrease in body weight to below 80% of the body weight at the start of administration.

## Claims

**1.** An $^{225}$Ac-labeled anti-CDH3 antibody comprising $^{225}$Ac binding to an anti-CDH3 antibody or to a recombinant antibody thereof, wherein
the anti-CDH3 antibody contains:

a heavy-chain variable region containing CDR1 that contains an amino acid sequence of SEQ ID NO:3, CDR2 that contains an amino acid sequence of SEQ ID NO:4, and CDR3 that contains an amino acid sequence of SEQ ID NO:5; and
a light-chain variable region containing CDR1 that contains an amino acid sequence of SEQ ID NO:6, CDR2 that contains an amino acid sequence of SEQ ID NO:7, and CDR3 that contains an amino acid sequence of SEQ ID NO:8.

**2.** The $^{225}$Ac-labeled anti-CDH3 antibody according to claim 1, wherein
the anti-CDH3 antibody contains:

a heavy-chain variable region containing an amino acid sequence of SEQ ID NO:9; and
a light-chain variable region containing an amino acid sequence of SEQ ID NO:10.

**3.** The $^{225}$Ac-labeled anti-CDH3 antibody according to claim 1 or 2, wherein
the anti-CDH3 antibody contains:

a heavy chain containing an amino acid sequence of SEQ ID NO:1; and
a light chain containing an amino acid sequence of SEQ ID NO:2.

**4.** The $^{225}$Ac-labeled anti-CDH3 antibody according to any one of claims 1 to 3, wherein
the anti-CDH3 antibody is a monoclonal antibody produced by an antibody-producing cell with Accession No. NITE BP-1040 or Accession No. NITE BP-1049.

**5.** The $^{225}$Ac-labeled anti-CDH3 antibody according to claim 1, wherein
the anti-CDH3 antibody or the recombinant antibody thereof binds to the $^{225}$Ac via a metal chelate.

**6.** The $^{225}$Ac-labeled anti-CDH3 antibody according to claim 5, wherein
the metal chelate is DOTA.

**7.** A cancer treatment agent comprising the $^{225}$Ac-labeled anti-CDH3 antibody according to claim 1.

**8.** The $^{225}$Ac-labeled anti-CDH3 antibody according to claim 1, which is for use in a cancer treatment.

**9.** Use of the $^{225}$Ac-labeled anti-CDH3 antibody according to claim 1 for a production of a cancer treatment agent.

**10.** A method for treating a cancer, comprising: administering the $^{225}$Ac-labeled anti-CDH3 antibody according to claim 1 to a subject in need thereof.

**11.** The cancer treatment agent according to claim 7, wherein a disease to be treated is a pancreatic cancer.

**12.** The cancer treatment agent according to claim 7, wherein a dose for administration to a human is 0.10 to 10 MBq/m$^2$.

**13.** The cancer treatment agent according to claim 7, wherein specific radioactivity of the $^{225}$Ac-labeled anti-CDH3 antibody is 0.01 to 5.0 MBq/mg.

**14.** The $^{225}$Ac-labeled anti-CDH3 antibody according to claim 8, wherein a disease to be treated is a pancreatic cancer.

**15.** The $^{225}$Ac-labeled anti-CDH3 antibody according to claim 8, wherein a dose for administration to a human is 0.10 to 10 MBq/m$^2$.

**16.** The $^{225}$Ac-labeled anti-CDH3 antibody according to claim 8, wherein specific radioactivity of the $^{225}$Ac-labeled anti-CDH3 antibody is 0.01 to 5.0 MBq/mg.

**17.** The use of the $^{225}$Ac-labeled anti-CDH3 antibody according to claim 9, wherein a disease to be treated is a pancreatic cancer.

**18.** The use of the $^{225}$Ac-labeled anti-CDH3 antibody according to claim 9, wherein a dose for administration to a human is 0.10 to 10 MBq/m$^2$.

**19.** The use of the $^{225}$Ac-labeled anti-CDH3 antibody according to claim 9, wherein specific radioactivity of the $^{225}$Ac-labeled anti-CDH3 antibody is 0.01 to 5.0 MBq/mg.

**20.** The method for treating a cancer according to claim 10, wherein a disease to be treated is a pancreatic cancer.

**21.** The method for treating a cancer according to claim 10, wherein a dose for administration to a human is 0.10 to 10 MBq/m$^2$.

**22.** The method for treating a cancer according to claim 10, wherein specific radioactivity of the $^{225}$Ac-labeled anti-CDH3 antibody is 0.01 to 5.0 MBq/mg.

[FIG. 1]

60.0
50.0
40.0
30.0
20.0
10.0
0.0
RADIOACTIVE CONCENTRATION (%ID/g)
5min
24h
48h
96h
168h
BLOOD
HEART
LUNG
LIVER
KIDNEY
SPLEEN
PANCREAS
BONE
TUMOR

[FIG. 2]

Vehicle
9
14
19
55
67
4000
3000
2000
1000
0
Tumor volume (mm3)
0
10
20
30
40
50
60
Days

[FIG. 3]

0.71 MBq/kg
18
32
37
64
65
350
250
150
50
-50
Tumor volume (mm3)
0
10
20
30
40
50
60
Days

[FIG. 4]

1.07 MBq/kg
2
15
43
44
48
350
250
150
50
-50
Tumor volume (mm3)
0
10
20
30
40
50
60
Days

[FIG. 5]

1.42 MBq/kg
3
20
34
57
63
350
250
150
50
-50
Tumor volume (mm3)
0
10
20
30
40
50
60
Days

[FIG. 6]

Vehicle
9
14
19
55
67
Relative body weight (%)
125
115
105
95
85
75
0
10
20
30
40
50
60
Days

[FIG. 7]

0.71 MBq/kg
18
32
37
64
65
Relative body weight (%)
125
115
105
95
85
75
0
10
20
30
40
50
60
Days

[FIG. 8]

1.07 MBq/kg
2
15
43
44
48
125
115
105
95
85
75
Relative body weight (%)
0
10
20
30
40
50
60
Days

[FIG. 9]

1.42 MBq/kg
3
20
34
57
63
125
115
105
95
85
75
Relative body weight (%)
0
10
20
30
40
50
60
Days

[FIG. 10]

Vehicle
9
11
12
15
19
2000
1500
1000
500
0
Tumor volume (mm3)
0
5
10
15
20
25
30
35
Days

[FIG. 11]

308 MBq/kg
1
8
16
18
20
350
250
150
50
-50
Tumor volume (mm3)
0
5
10
15
20
25
30
35
Days

[FIG. 12]

Vehicle
9
11
12
15
19
Relative body weight (%)
125
115
105
95
85
75
0
5
10
15
20
25
30
35
Days

[FIG. 13]

308 MBq/kg
1
8
16
18
20
Relative body weight (%)
125
115
105
95
85
75
0
5
10
15
20
25
30
35
Days

## INTERNATIONAL SEARCH REPORT

International application No. **PCT/JP2024/038102**

**A. CLASSIFICATION OF SUBJECT MATTER**

***C07K 16/28***(2006.01)i; ***A61K 39/395***(2006.01)i; ***A61K 51/10***(2006.01)i; ***A61P 35/00***(2006.01)i; ***C12N 5/20***(2006.01)i; ***C12N 15/13***(2006.01)i; ***C12P 21/08***(2006.01)i; *A61K 103/40*(2006.01)n
FI: C07K16/28 ZNA; C12N15/13; A61K39/395 T; A61K51/10 100; A61P35/00; C12N5/20; C12P21/08; A61K103:40

According to International Patent Classification (IPC) or to both national classification and IPC

**B. FIELDS SEARCHED**

Minimum documentation searched (classification system followed by classification symbols)

C07K16/28; A61K39/395; A61K51/10; A61P35/00; C12N5/20; C12N15/13; C12P21/08; A61K103/40

Documentation searched other than minimum documentation to the extent that such documents are included in the fields searched

Published examined utility model applications of Japan 1922-1996
Published unexamined utility model applications of Japan 1971-2024
Registered utility model specifications of Japan 1996-2024
Published registered utility model applications of Japan 1994-2024

Electronic data base consulted during the international search (name of data base and, where practicable, search terms used)

JSTPlus/JMEDPlus/JST7580 (JDreamIII); CAplus/REGISTRY/MEDLINE/EMBASE/BIOSIS (STN)

**C. DOCUMENTS CONSIDERED TO BE RELEVANT**

| Category* | Citation of document, with indication, where appropriate, of the relevant passages | Relevant to claim No. |
|---|---|---|
| Y | SUBBIAH, Vivek et al. Phase I Study of P-cadherin-targeted Radioimmunotherapy with 90Y-FF-21101 Monoclonal Antibody in Solid Tumors. Clinical Cancer Research. 15 November 2020, vol. 26, no. 22, pp. 5830-5842<br>p. 5830, abstract, p. 5837, fig. 3 | 1-22 |
| Y | WO 2011/099524 A1 (FUJIFILM RI PHARMA CO., LTD.) 18 August 2011 (2011-08-18)<br>claims | 1-22 |
| Y | SUDO, Hitomi et al. Preclinical Evaluation of Podoplanin-Targeted Alpha-Radioimmunotherapy with the Novel Antibody NZ-16 for Malignant Mesothelioma. Cells. vol. 10, no. 2503, pp. 1-15<br>abstract, results 3.4, fig. 3 | 1-22 |
| A | JP 2016-56165 A (FUJITA GAKUEN) 21 April 2016 (2016-04-21) | 1-22 |

☐ Further documents are listed in the continuation of Box C. ☑ See patent family annex.

* Special categories of cited documents:
"A" document defining the general state of the art which is not considered to be of particular relevance
"D" document cited by the applicant in the international application
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **25 November 2024** | **03 December 2024** |

| Name and mailing address of the ISA/JP | Authorized officer |
|---|---|
| **Japan Patent Office (ISA/JP)**<br>**3-4-3 Kasumigaseki, Chiyoda-ku, Tokyo 100-8915**<br>**Japan** | Telephone No. |

Form PCT/ISA/210 (second sheet) (July 2022)

**INTERNATIONAL SEARCH REPORT**

International application No.
**PCT/JP2024/038102**

**Box No. I Nucleotide and/or amino acid sequence(s) (Continuation of item 1.c of the first sheet)**

1. With regard to any nucleotide and/or amino acid sequence disclosed in the international application, the international search was carried out on the basis of a sequence listing:
   a. ☑ forming part of the international application as filed.
   b. ☐ furnished subsequent to the international filing date for the purposes of international search (Rule 13*ter*.1(a)),
      ☐ accompanied by a statement to the effect that the sequence listing does not go beyond the disclosure in the international application as filed.

2. ☐ With regard to any nucleotide and/or amino acid sequence disclosed in the international application, this report has been established to the extent that a meaningful search could be carried out without a WIPO Standard ST.26 compliant sequence listing.

3. Additional comments:

**INTERNATIONAL SEARCH REPORT**
**Information on patent family members**

International application No.
**PCT/JP2024/038102**

| Patent document cited in search report | Publication date (day/month/year) | Patent family member(s) | Publication date (day/month/year) |
|---|---|---|---|
| WO 2011/099524 A1 | 18 August 2011 | EP 2535358 A1<br>claims<br>US 2013/0071324 A1<br>US 2014/0328754 A1<br>JP 2014-12731 A | |
| JP 2016-56165 A | 21 April 2016 | (Family: none) | |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 5593560 B
- JP 5380553 B


### Non-patent literature cited in the description


- *Clinical Cancer Research*, 15 November 2020, vol. 26 (22), 5830-5842
- *Chemistry Society Reviews*, 2014, vol. 43, 260-290
- *J Basic Clin Pharm.*, March 2016, vol. 7 (2), 27-31